(19) Europäisches Patentamt
European Patent Office
Office européen des brevets

(11) Numéro de publication: **0 092 454 B1**

# (12) FASCICULE DE BREVET EUROPEEN

(45) Date de publication du fascicule du brevet: **27.11.85**

(51) Int. Cl.[4]: **C 07 C 119/048,** C 07 C 118/00 // C08G18/76

(21) Numéro de dépôt: **83400658.7**

(22) Date de dépôt: **30.03.83**

(54) **Isocyanates aromatiques halogénés ou leurs mélanges, à structure diphénylméthane.**

(30) Priorité: **16.04.82 FR 8206562**

(43) Date de publication de la demande: **26.10.83 Bulletin 83/43**

(45) Mention de la délivrance du brevet: **27.11.85 Bulletin 85/48**

(84) Etats contractants désignés: **AT BE CH DE FR GB IT LI LU NL SE**

(56) Documents cités:
**EP - A - 0 046 917**
**EP - B - 0 024 665**
**DE - A - 2 452 715**
**FR - A - 1 447 964**

(73) Titulaire: **ATOCHEM, 12/16, allée des Vosges, F-92400 Courbevoie (FR)**

(72) Inventeur: **Kervennal, Jacques, 134, rue Docteur Locard, F-69005 Lyon (FR)**
Inventeur: **Mathais, Henri, 87, chemin de l'Indiennerie, F-69370 Saint Didier au Mont d'Or (FR)**

**EP 0 092 454 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne de nouveaux isocyanates aromatiques halogénés et leurs mélanges ayant pour squelettes hydrocarbonés des structures dissymétriques méthyldiphénylméthane ou polyméthyldiphénylméthane substituées par un ou plusieurs atomes d'hydrogène. Ces isocyanates et leurs mélanges sont utilisables dans la fabrication de polyuréthannes et particulièrement de mousses ignifugées.

Des isocyanates de structure aromatique hydrocarbonée diphénylméthane sont déjà synthétisés industriellement; il s'agit essentiellement des dérivés du diphénylméthane diisocyanate (MDI), commercialisés, soit sous forme brute, c'est-à-dire sous forme d'un mélange de diisocyanates et de polyisocyanates, soit sous forme pure après distillation. Dans ce cas, le mélange d'isomères, contenant essentiellement du diisocanate-4,4' diphénylméthane est solide à température ordinaire et présente un point de fusion compris entre 30 et 40° C. Pour remédier à cet inconvénient ont été essayés les isomères de MDT autres que le 4,4' comme ceux décrits dans le EP-B N° 0024665. Ont également été essayés en remplacement du MDI, des mélanges d'isomères de polyisocanate à structure diphénylméthane possédant plus de deux fonctions −NCO dans leur molécule comme dans le cas du EP-A N° 0046917. Les synthèses de polyurétahnnes s'effectuent par réaction des isocyanates sur des polyols. Des qualités d'ignifugation peuvent être apportées aux polymères par l'utilisation de polyols halogénés et/ou d'additifs halogénés. Les isocyanates de l'invention, fluides à la température ambiante, présentant eux-mêmes un ou plusieurs atomes d'halogène sur les cycles aromatiques, sont ainsi particulièrement intéressants pour la préparation de polyuréthanne ayant une bonne résistance au feu.

Les isocyanates à structures symétriques halogénés dérivés du diisocyanate symétrique 4,4' diphénylméthane sont connus du FR-A N° 1447964. Toutefois la commercialisation de tels produits n'est que très limitée, leur fabrication à partir d'aniline, dont la toxicité est bien connue, étant des plus restreintes. Parmi les isocyanates halogénés à structure phénylméthane on peut encore citer le bis(diisocyanatobenzyl)chlorbenzyle du DE-A N° 2453715 qui contient quatre fonctions −NCO par molécule dont deux sur chaque noyau benzyl. De tels produits, polymérisés, ne peuvent conduire qu'à des polyuréthannes fortement réticulés n'ayant aucun rapport avec ceux obtenus à partir d'isocyanates ne possédant qu'une seule fonction −NCO par noyau benzyle.

Les structures isocyanates selon l'invention répondent à la formule générale suivante:

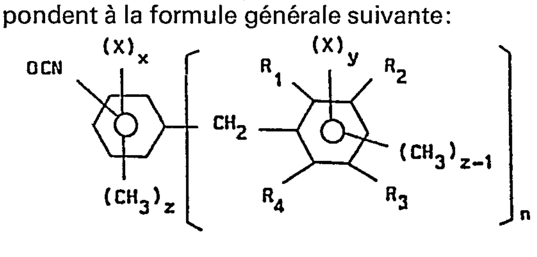

avec
$X = Cl, Br$,
$n = 1, 2$,
$x = 0, 1, 2$,
$y = 1, 2$,
$z = 1, 2$,
l'un des groupements $R_1$, $R_2$, $R_3$, $R_4$ étant un groupement NCO, les autres étant des atomes d'hydrogène en fonction des valeurs et positions de $(X)_y$ et $(CH_3)_{z-1}$.

La synthèse de ces isocyanates fait appel à trois étapes réactionnelles successives à partir d'un mélange d'hydrocarbures aromatiques halogénés: une de nitratation, une d'hydrogénation et une de phosgénation.

Les structures hydrocarbonées halogénées sont préparées par condensation de type Friedel et Crafts, en présence de catalyseurs utilisés classiquement dans ces réactions, de chlorure de benzyle halogéné éventuellement substitué par un groupement méthyle, avec du toluène ou du xylène éventuellement halogéné et placé en excès. On obtient ainsi différents mélanges d'isomères aromatiques halogénés. Suivant les conditions opératoires, en réglant l'excès de toluène ou de xylène éventuellement halogéné, on obtient essentiellement des produits de condensation à deux noyaux aromatiques, mais il se forme également des produits à trois noyaux, l'ensemble répondant à la formule générale suivante:

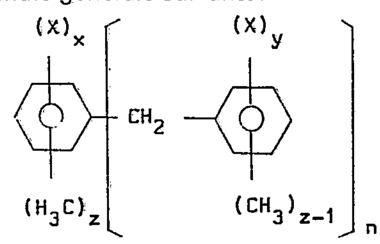

avec
$X = Cl$ ou $Br$,
$x = 0$ à $2$,
$y = 1$ ou $2$,
$z = 1$ ou $2$,
$n = 1$ ou $2$.

Il est possible de séparer, par distillation, les isomères à deux noyaux du milieu de réaction, cependant, il peut être économiquement intéressant d'effectuer la synthèse des isocyanates sur le mélange réactionnel brut. En pratique, les produits de réaction contiennent entre 70 et 95% de condensats diarylés.

Parmi les hydrocarbures aromatiques servant de base à l'invention, la synthèse des isomères de (chlorobenzyle) toluène peut se faire de différentes façons, mais il est préférable de faire réagir le chlorure de chlorobenzyle, obtenu par chloration photochimique ménagée de monochlorotoluène, sur le toluène en présence de catalyseurs utilisés dans de telles réactions; en effet, le toluène est dans ce cas la seule matière première hydrocarbonée. Les isomères de (bromobenzyle) toluène sont synthétisés de la même façon à partir du chlorure de bromobenzyle.

La synthèse d'autres hydrocarbures aromatiques de structures (polychloro)méthyldiphénylméthane ou (polychloro) (polyméthyl)diphénylméthane est connue et décrite dans le brevet français N° 2432199 et le certificat d'addition N° 2449954.

Les deux étapes que constituent la nitration puis l'hydrogénation conduisent aux amines conformes aux structures isocyanates obtenues selon l'invention:

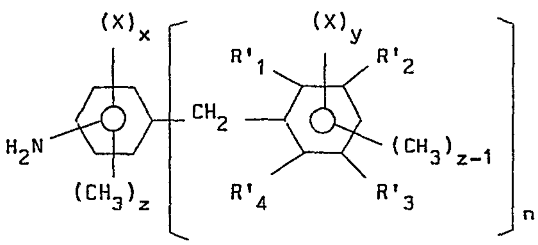

avec
X = Cl, Br,
n = 1, 2,
x = 0, 1, 2,
y = 1, 2,
z = 1, 2,
l'un des groupements $R'_1$, $R'_2$, $R'_3$, $R'_4$ étant un groupement $NH_2$, les autres étant des atomes d'hydrogène.

On utilise pour la nitration un mélange d'acide nitrique et d'acide sulfurique concentrés à au moins 85% en poids et de préférence au moins 95%. L'acide nitrique peut être placé en quantités stœchiométriques par rapport au composé aromatique. On utilise donc une mole d'acide par noyau aromatique à nitrer. Il est cependant avantageux d'opérer en présence d'un excès d'acide nitrique pouvant aller jusqu'à 20% par rapport à la stœchiométrie. L'acide sulfurique peut être utilisé en quantité équimolaire par rapport à l'acide nitrique, mais on peut le placer en excès ou en défaut. La réaction de nitration s'effectue entre 0° C et la température d'ébullition des mélanges, habituellement entre 0 et 50° C, le composé aromatique étant de préférence solubilisé dans un solvant tel que le chlorure de méthylène. La phase organique, supérieure, est ensuite séparée des acides, neutralisée et évaporée à sec. Il est recommandé d'opérer, sous atmosphère d'azote, dans un réacteur muni de moyens d'agitation efficace et de régulation de température.

Le mélange brut obtenu peut être hydrogéné tel quel. Cependant, dans le cas où les composés diarylés sont distillés, il est possible d'en isoler les isomères majoritaires en solubilisant le mélange dans le minimum de chlorure de méthylène et en les précipitant à l'acide d'éther ou de méthanol.

La deuxième étape constitue l'hydrogénation des dérivés nitrés en amines correspondantes; elle peut être chimique, mais il est préférable d'opérer sous pression d'hydrogène, dans un réacteur résistant à la pression muni d'une agitation et possédant les moyens de contrôle et régulation classiques, en présence d'un catalyseur à base de nickel, palladium, platine, ruthénium et autres. Dans ce cas, on utilise un réacteur d'hydrogénation permettant de travailler sous des pressions pouvant atteindre 100 bars. La réaction peut s'effectuer sans solvant à une température où le dérivé nitré est fondu, ou dans des solvants classiques d'hydrogénation tels que les alcools, le dioxane, les éthers de l'éthylèneglycol et autres.

On utilise préférentiellement un catalyseur constitué de palladium déposé sur support à des teneurs comprises entre 1 et 10%, ce qui permet d'opérer à des températures comprises entre 30 et 100° C et des pressions de 20 à 50 bars. Le rapport molaire $\frac{\text{dérivé dinitré}}{\text{palladium}}$ n'est pas impérativement fixé, mais il est de préférence compris entre 200 et 3000. Après réaction et filtration du catalyseur, le solvant éventuellement utilisé est évaporé et le mélange des isomères de diamines obtenues peut être utilisé tel quel ou distillé.

La troisième étape fait appel à des techniques classiques de phosgénation dans un réacteur muni d'une agitation et surmonté d'un réfrigérant. Le mélange d'amines est placé dans un solvant aromatique chloré tel que le monochlorobenzène ou l'orthodichlorobenzène contenant la quantité nécessaire de phosgène, et maintenant la température à 0° C. On chauffe ensuite la suspension progressivement; le mélange réactionnel s'homogénéise entre 45 et 60° C. On continue d'élever lentement la température jusqu'au point d'ébullition du mélange, puis on distille le solvant de façon à récupérer les isocyanates formés. Ces derniers peuvent être distillés, s'ils sont suffisamment volatils, ou utilisés tels quels. Le mélange d'amines est de préférence placé dans le solvant à une concentration de 5 à 20% et le phosgène est introduit en quantités stœchiométriques ou en léger excès. Une autre technique consiste à introduire conjointement dans le réacteur une solution portée au-dessus de 100° C des amines dans un solvant aromatique chloré et un courant de phosgène solubilisé dans le même solvant qu'on amène à 120° C ou plus. La réaction est alors pratiquement immédiate.

Les isocyanates ainsi obtenus peuvent conduire, selon les techniques connues, à des polyuréthannes possédant de bonnes propriétés, et notamment une résistance au feu améliorée.

Les analyses sur les dérivés dinitrés et les amines ont été effectuées par chromatographie en phase gazeuse. Dans le cas des produits de nitration, il a été utilisé une colonne en inox d'1/8", longueur de 1,5 m, emplie d'un support Chromosorb W. 80-100 mesh H.P., commercialisé par la firme Johns Manville, imprégné à 2% de phase silicone XE.60, „Applied Science Laboratories, Inc."; lors de l'injection, la colonne est à 100° C et au bout de 5 min est portée à 220° C. Il a également été utilisé une colonne en pyrex de diamètres intérieur et extérieur 3 et 6 mm, de longueur 2 m, remplie de phase SP 2250 imprégnée à 3% sur support Supelcoport 100-120 mesh (Supelco) sur laquelle a été effectuée une programmation linéaire de température à 4° C/min de 180 à 280° C.

Dans le cas des amines, a été utilisée une colonne en verre de 2 m, emplie de support chromosorb W.N.A.W. 60-80 mesh, Johns Manville, imprégnée à 5% de KOH et 5% d'Apiézon N – Société

Apiézon Products Limited – en opérant en isotherme à 220° C.

Les identifications des produits de nitration ou d'hydrogénation ont été faites par couplage de la chromotographie en phase gazeuse avec la spectrométrie de masse. Les isomères principaux ont été dans certains cas isolés par piégage en chromatographies gazeuse et/ou liquide et identifiés par résonance magnétique nucléaire du $^{13}C$ et du $^{1}H$.

Les teneurs en fonctions isocyanates ont été évaluées par dosage chimique à la dibutylamine.

*Exemple 1:*

On dissout 22 g (0,1 mol) des isomères de (chlorobenzyl) toluène distillés, préparés par condensation de Friedel et Crafts du chlorure de chlorobenzyle sur le toluène, dans 40 ml de chlorure de mtéyhlène. En maintenant la température autour de 10° C sous agitation, on verse en ½ h un mélange de 15,5 g d'acide nitrique de 98,7% et de 20 ml d'acide sulfurique à 96%. Dès la fin de l'addition, on porte à reflux pendant 3½ h en maintenant l'agitation, puis refroidit. Deux phases se séparent, l'une inférieure acide, l'autre supérieure organique. On extrait la phase organique et lave deux fois les acides avec du chlorure de méthylène. On réunit les différentes fractions organiques, neutralise sur carbonate de potassium et évapore le solvant. On récupère ainsi 30,5 g d'un produit jaune clair visqueux dont l'analyse par chromatographie gazeuse couplée avec la spectrométrie de masse met en évidence les isomères de (nitrochlorobenzyle)-nitrotoluène. Le constituant principal du mélange est le dinitro-3,3' méthyl-4 chloro-4' diphénylméthane de formule:

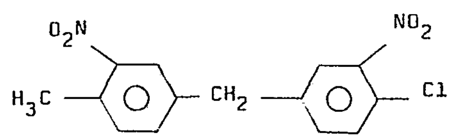

On place 20 g de ce mélange d'isomères dans l'autoclave précédemment décrit et ajoute 200 ml de méthanol ainsi que 0,2 g de catalyseur constitué de palladium déposé sur charbon à 5%. Après avoir balayé le réacteur avec de l'azote, on y introduit 40 bars d'hydrogène et le chauffe, sous agitation, jusqu'à 60° C. Après 3 h 20 de réaction, on laisse refroidir, décomprime et récupère le mélange réactionnel qu'on filtre pour éliminer le catalyseur. Le méthanol est évaporé à sec et on obtient 16 g d'un produit brun-foncé très épais. L'analyse par couplage de la chromatographie gazeuse avec la spectrométrie de masse montre qu'il contient les isomères d'(aminochlorobenzyle)aminotoluène. On introduit 12 g de ceux-ci dans une solution de 29 g de phosgène dans 100 ml d'orthodichlorobenzène en maintenant la température à 0° C. On laisse ensuite remonter à température ambiante, puis on chauffe à 45° C pendant 1 h, à 60° C pendant 1 h et à 100° C pendant ½ h. En faisant passer un courant d'azote, on amène enfin la température à 140° C pendant 2 h afin de chasser le phosgène en excès et l'acide chlorhydrique formé. Puis on distille le solvant sous vide après filtration de la solution et recueille 13 g de mélange d'isocyanates (teneur en NCO: 5 Eq/kg) dont la distillation conduit à un liquide jaune constitué d'un mélange de diisocyanate du (chlorobenzyle)toluène (teneur en NCO: 2 fonctions par mole), correspondant à la formule générale revendiquée dans laquelle X = Cl, x = 0, y = 1, z = 1, n = 1.

*Exemple 2:*

On dissout 138 g du mélange d'isomères de (chlorobenzyl)toluène préparés dans l'exemple 1 dans 240 ml de chlorure de méthylène. En opérant de la même façon que dans l'exemple 1, on additionne un mélange de 93 g d'acide nitrique à 98,3% et de 120 ml d'acide sulfurique à 96%. On chauffe à reflux pendant 4 h, puis refroidit et extrait la phase organique. On évapore le solvant jusqu'à la limite de solubilité des isomères de dinitration, puis on ajoute du méthanol et précipite ainsi un solide blanc qu'on filtre et lave au méthanol. On récupère après séchage 21,5 g de solide blanc de point de fusion 183° C. L'analyse par chromatographie gazeuse met en évidence la présence nettement majoritaire d'un isomère de dinitro(chlorobenzyl)-toluène que l'étude en résonance magnétique nucléaire identifie comme étant le dinitro-3,3' méthyl-4 chloro-4' diphénylméthane:

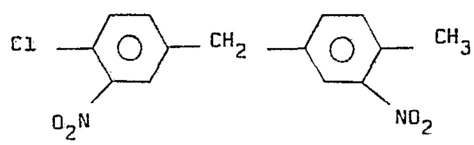

Le filtrat méthanolique contient le reste de cet isomère majoritaire ainsi que les autres isomères de dinitration.

20 g du précipité sont hydrogénés suivant le mode opératoire décrit dans l'exemple 2 à l'aide de 200 ml de méthanol et 0,4 g de catalyseur Pd sur charbon à 5%. On récupère, après évaporation du méthanol, 16 g de produit brun foncé, pâteux, constitué principalement de diamino-3,3' méthyl-4 chloro-4' diphénylméthane.

On phosgène, suivant le mode opératoire exposé dans l'exemple 1, 10 g de ce mélange d'amines. On récupère ainsi 12 g de produit brun pâteux (teneur en NCO = 6 Eq/kg) qui, distillé sous pression réduite de 2 mm de mercure, conduit à un solide jaune clair, pâteux, entièrement fondu à 64° C et contenant environ 66% de diisocyanate-3,3' méthyl-4 chloro-4' diphénylméthane (teneur en NCO: 2 Eq/mol).

*Exemple 3:*

Dans un réacteur agité de 250 ml, on place, sous atmosphère d'azote, 25,1 g (0,1 mol) d'un mélange d'isomères de (chlorobenzyl)chlorotoluène synthétisés à partir de chlorotoluène selon la description faite dans le brevet français N° 2432199. On ajoute 40 ml de chlorure de méthylène et introduit en ½ h entre 5 et 15° C un mélange de 13,5 g d'acide nitrique à 98,3% et 20 ml d'acide sulfurique à 96%. On chauffe ensuite à 42° C pendant 3½ h, puis refroidit et laisse décanter. On récupère la phase organique supérieure et lave par du chlorure

de méthylène la phase acide inférieure. Les extraits organiques de soutirage et de lavage sont mélangés, neutralisés avec du carbonate de potassium et séchés à l'aide de sulfate de sodium. On obtient une solution limpide de couleur orangée qu'on évapore à sec. On recueille ainsi 32 g de liquide orangé très visqueux correspondant au mélange d'isomères de (nitrochlorobenzyl)nitrochlorotoluène. Les constituants principaux du mélange sont le dinitro-2,3' méthyl-5 dichloro-4,2' diphénylméthane, de formule:

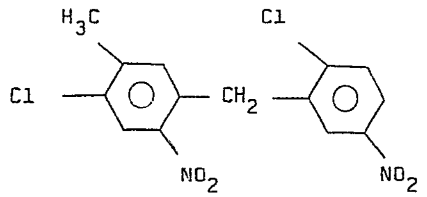

le dinitro-2,3' méthyl-5 dichloro-4,4' diphénylméthane, de formule:

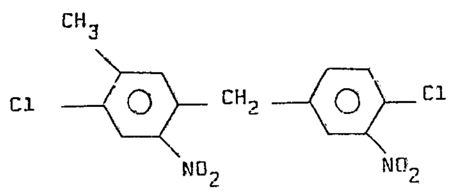

le dinitro-3,3' méthyl-4 dichloro-5,4' diphénylméthane, de formule:

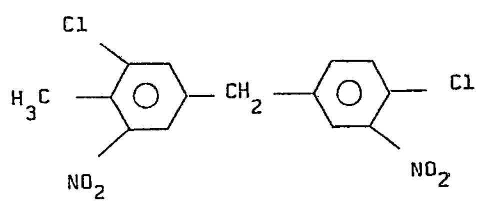

le dinitro-3,3' méthyl-5 dichloro-4,4' diphénylméthane, de formule:

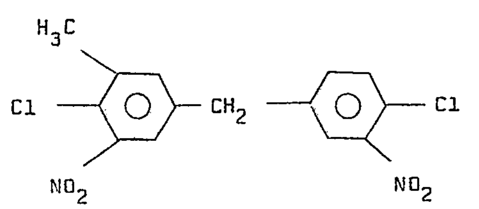

On hydrogène 30 g de ce liquide, dans un autoclave en acier inox muni d'une agitation magnétique, en présence de 0,3 g de catalyseur palladium sur charbon à 5% (Engelhard) et 300 ml de méthanol. En opérant à 35 bars et 50° C, la réaction dure 1½ h, puis on décharge le réacteur et récupère après évaporation du solvant 24,5 g d'un solide orangé de point de fusion 45-47° C. L'analyse par chromatographie gazeuse montre qu'il s'agit des isomères de la diamine attendue, répondant à la formule générale de l'invention dans laquelle X= Cl, x = 1, y = 1, z = 1, n = 1.

On phosgène, conformément à la méthode précédemment décrite, le mélange d'amines. On obtient, après réaction et évaporation du solvant, 29 g de mélange brut d'isocyanates (teneur en NCO: 5,2 Eq/kg) qui, distillé sous pression réduite de 2 mm de mercure, conduit à un liquide jaune visqueux formé d'isomères de diisocyanates attendus (teneur en NCO: 2 fonctions par mole).

*Exemple 4:*

27,9 g (0,1 mol) des isomères de (chloroparaméthylbenzyle)chloroparaxylène préparés par réaction de condensation de Friedel et Crafts de chlorure de chloroparaméthylbenzyle sur le chloroparaxylène, sont dissous dans 40 ml de chlorure de méthylène. On maintient la température autour de 10° C, agite et introduit en ½ h un mélange de 15,5 g d'acide nitrique à 98,7% et de 20 ml d'acide sulfurique à 96%. On porte à reflux pendant 3½ h, puis refroidit. On récupère la phase organique supérieure et lave les acides avec du chlorure de méthylène. Les phases organiques sont rassemblées et traitées avec du carbonate de potassium, puis le solvant est évaporé. On récupère ainsi 36 g de produits de dinitration correspondant à la formule suivante:

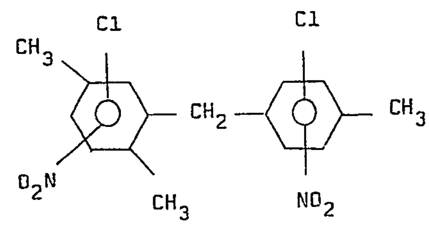

On hydrogène 20 g de ce mélange suivant le mode opératoire décrit dans l'exemple 1, avec 0,2 l de méthanol et 0,4 g de catalyseur Pd/charbon à 5%. On récupère, après distillation du méthanol, 16,5 g d'un produit cristallisé rougeâtre de point de fusion 73° C, correspondant essentiellement aux isomères d'(aminochloroparaméthylbenzyle)-aminochloroparaxylène.

15 g de ce mélange sont phosgénés selon la méthode décrite dans l'exemple 1. On récupère, après filtration et distillation de l'orthodichlorobenzène, 15 g de produit brut brun de point de fusion 49° C, contenant un mélange des isocyanates attendus (teneur en NCO: 4,4 Eq/kg), en accord avec la formule générale donnée dans le descriptif où X = Cl, x = 1, y = 1, z = 2, n = 1.

*Exemple 5:*

La condensation de Friedel et Crafts du chlorure de chlorobenzyle sur le toluène conduit, outre à la formation de composés diarylés, traités dans l'exemple 1, à des produits de condensation à trois noyaux aromatiques. Les composés à deux noyaux, correspondant aux isomères de (chlorobenzyle)toluène, peuvent être séparés par distillation, les dérivés triarylés restant en pied de colonne.

On nitre, selon le mode opératoire décrit dans l'exemple 1, 34 g d'un mélange formé essentiellement d'isomères de bis(chlorobenzyl)toluène, en utilisant 23,2 g d'acide nitrique à 98,7% et 20,2 ml d'acide sulfurique à 96%. On recueille, en fin de

réaction, 48 g d'un produit rougeâtre très visqueux dont on hydrogène 20 g, placés dans l'autoclave avec 200 ml de métahnol et 0,6 g de catalyseur palladium sur charbon à 5% (Engelhard), en opérant sous 40 bars d'hydrogène et à 65° C. On recueille ainsi 14,5 g d'un mélange brut d'amines, de point de fusion 127° C, qu'on fait réagir avec 33 g de phosgène suivant la description faite dans l'exemple 1. Après filtration de la solution de réaction dans l'orthodichlorobenzène, on évapore le solvant et récupère 13 g d'un produit brut fondant à 124° C et contenant les isomères isocyanates conformes à la formule générale de l'invention avec X = Cl, x = 0, y = 1, z = 1 et n = 2 (teneur en NCO: 4,4 fonctions par kg).

*Exemple 6:*

En opérant comme dans l'exemple 1, on nitre, avec 18,7 g d'acide nitrique à 98,7% et 21,5 ml d'acide sulfurique à 96%, 23,5 g d'un mélange brut de condensation de Friedel et Crafts du chlorure de chlorobenzène sur le toluène, contenant 85% en poids de dérivés diarylés et 15% de dérivés essentiellement triarylés. On obtient, après réaction, 32 g d'un produit de nitration mi-solide, mi-visqueux, dont on hydrogène 20 g dans 200 ml de méthanol, en présence de 0,8 g de catalyseur palladium sur charbon à 5% (Engelhard) en opérant à 65° C sous 40 bars d'hydrogène. Après réaction et évaporation du méthanol, on recueille 15 g d'un mélange brut d'amines qu'on fait réagir avec 36,2 g de phosgène conformément à la description de l'exemple 1. Après filtration de la solution de réaction dans l'orthodichlorobenzène, on évapore le solvant et récupère 13,8 g d'un produit brun pâteux qui prend en masse au refroidissement et dont l'examen en spectrométrie de masse montre qu'il contient des isomèes d'isocyanates diarylés et triarylés correspondant à la formule brute du descriptif dans laquelle X = Cl, x = 0, y = 1, z = 1 et n = 1 et 2. Ce mélange d'isocyanates, qui peut être facilement coulé à 60° C, a une teneur en fonctions NCO de 5,4 Eq/kg.

*Exemple 7:*

On nitre, à l'aide de 15,5 g d'acide nitrique à 98,7% et de 20 ml d'acide sulfurique à 96% dans les conditions décrites dans l'exemple 1, 32 g de mélange d'isomères de (dichlorobenzyle)-dichlorotoluène préparé par condensation de Friedel et Crafts de chlorure de dichlorobenzyle sur le dichlorotoluène. On récupère 39 g d'un mélange très visqueux de couleur orangée.

On hydrogène 30 g de celui-ci en présence de 0,3 g de catalyseur palladium sur charbon à 5% (Engelhard) dans 300 ml de méthanol, à 60° C sous 40 bars, ce qui conduit à 25 g d'un mélange solide d'amines de couleur brun foncé qu'on phosgène dans les conditions décrites précédemment. On obtient finalement 28 g de mélange brut d'isocyanates (teneur en NCO: 4,1 Eq/kg) correspondant à la formule générale donnée dans le descriptif, dans laquelle X = Cl, x = 2, y = 2, z = 1, n = 1.

**Revendications**

1. Isocyanates aromatiques halogénés à structure dissymétrique méthyldiphénylméthane ou polyméthyldiphénylméthane de formule:

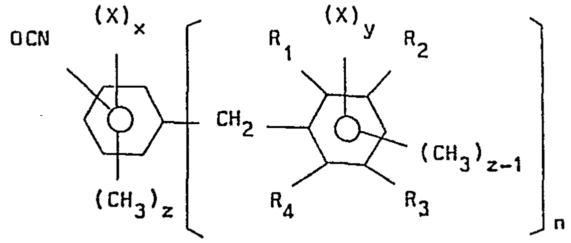

avec
X = Cl, Br,
n = 1, 2,
x = 0, 1, 2,
y = 1, 2,
z = 1,2,

l'un des groupements $R_1$, $R_2$, $R_3$, $R_4$ étant un groupement NCO, les autres étant des atomes d'hydrogène en fonction des valeurs et positions de $(X)_y$ et $(CH)_{3_{z-1}}$.

2. Isocyanates selon la revendication 1, caractérisés en ce qu'il s'agit des isomères de (chlorobenzylisocyanate)toluèneisocyanate.

3. Isocyanates selon la revendication 1, caractérisés en ce qu'il s'agit des isomères de (chlorobenzylisocyanate)chlorotoluèneisocyanate.

4. Isocyanates selon la revendication 1, caractérisés en ce qu'il s'agit des isomèdes de (chloroparaméthylbenzylisocyanate)chloroparaxylène-isocyanate.

5. Isocyanates selon la revendication 1, caractérisés en ce qu'il s'agit des isomères de bis(chlorobenzylisocyanate)toluèneisocyanate.

6. Isocyanates selon la revendication 1, caractérisés en ce qu'il s'agit de mélanges d'isomères de (chlorobenzylisocyanate)toluèneisocyanate et d'isomères de bis(chlorobenzylisocyanate)-toluèneisocyanate.

7. Procédé de fabrication d'isocyanates aromatiques halogénés à structure dissymétrique diphénylméthane ou polyméthyldiphénylméthane selon les revendications 1 à 6, caractérisé en ce que les composés de formule:

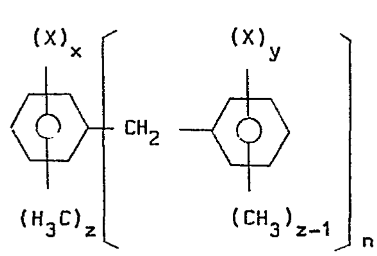

où X représente un atome de chlore ou de brome avec
x = 0 à 2,
y = 1 ou 2,
z = 1 ou 2,
n = 1 ou 2,

sont successivement nitrés, hydrogénés puis phosgénés.

8. Procédé selon la revendication 7, caractérisé en ce que les composés à structures hydrocarbonées halogénées sont obtenus par condensation d'un chlorure de benzyle halogéné avec du toluène ou du xylène.

9. Procédé selon la revendication 8, caractérisé en ce que le chlorure de benzyle halogéné est substitué par un groupement méthyle.

10. Procédé selon la revendication 8, caractérisé en ce que le toluène ou le xylène est chloré ou bromé.

11. Procédé selon l'une des revendications 7 à 10, caractérisé en ce que la nitration s'effectue au moyen d'un mélange d'acide nitrique et d'acide sulfurique de concentrations supérieures à 85%.

12. Procédé selon l'une des revendications 7 à 11, caractérisé en ce que l'hydrogénation s'effectue en présence de palladium comme catalyseur.

## Patentansprüche

1. Halogenierte aromatische Isocyanate mit asymmetrischer Methyldiphenylmethan- oder Polymethylphenylmethan-Struktur der Formel

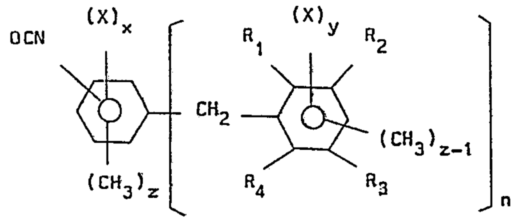

mit

x = Cl, Br,
n = 1, 2,
x = 0, 1, 2,
y = 1, 2,
z = 1, 2,

wobei eine der Gruppen $R_1$, $R_2$, $R_3$, $R_4$ eine NCO-Gruppe ist und die anderen Wasserstoffatome sind in Abhängigkeit von Wert und Stellung von $(X)_y$ und $(CH_3)_{z-1}$.

2. Isocyanate nach Anspruch 1, dadurch gekennzeichnet, dass es sich um Isomere von (Chlorbenzylisocyanat)toluolisocyanat handelt.

3. Isocyanate nach Anspruch 1, dadurch gekennzeichnet, dass es sich um Isomere von (Chlorbenzylisocyanat)chlortoluolisocyanat handelt.

4. Isocyanate nach Anspruch 1, dadurch gekennzeichnet, dass es sich um Isomere von (Chlorparamethylbenzylisocyanat)chlorparaxylolisocyanat handelt.

5. Isocyanate nach Anspruch 1, dadurch gekennzeichnet, dass es sich um Isomere von Bis(chlorbenzylisocyanat)toluolisocyanat handelt.

6. Isocyanate nach Anspruch 1, dadurch gekennzeichnet, dass es sich um Isomerengemische von (Chlorbenzylisocyanat)toluolisocyanat und von Bis(chlorbenzylisocyanat)toluolisocyanat handelt.

7. Verfahren zur Herstellung von halogenierten aromatischen Isocyanaten mit asymmetrischer Diphenylmethan- oder Polymethyldiphenylmethan-Struktur nach den Ansprüchen 1 bis 6, dadurch gekennzeichnet, dass die Verbindungen der Formel

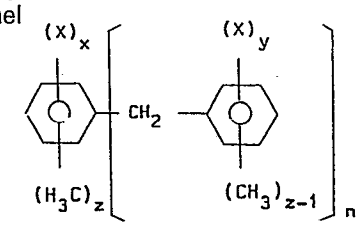

in der X ein Chlor- oder Bromatom bedeutet mit

x = 0 bis 2,
y = 1 oder 2,
z = 1 oder 2,
n = 1 oder 2,

zuerst nitriert, dann hydriert und anschliessend mit Phosgen umgesetzt werden.

8. Verfahren nach Anspruch 7, dadurch gekennzeichnet, dass die Verbindungen mit halogenierter Kohlenwasserstoffstruktur durch Kondensation eines halogenierten Benzylchlorids mit Toluol oder Xylol erhalten werden.

9. Verfahren nach Anspruch 8, dadurch gekennzeichnet, dass das halogenierte Benzylchlorid durch eine Methylgruppe substituiert ist.

10. Verfahren nach Anspruch 8, dadurch gekennzeichnet, dass das Toluol oder Xylol chloriert oder bromiert ist.

11. Verfahren nach einem der Ansprüche 7 bis 10, dadurch gekennzeichnet, dass man die Nitrierung mit einer Mischung aus Salpetersäure und Schwefelsäure einer Konzentration über 85% durchführt.

12. Verfahren nach einem der Ansprüche 7 bis 11, dadurch gekennzeichnet, dass die Hydrierung in Gegenwart von Palladium als Katalysator durchgeführt wird.

## Claims

1. Halogenated aromatic isocyanates having an unsymmetrical methyldiphenylmethane or polymethyldiphenylmethane structure of formula:

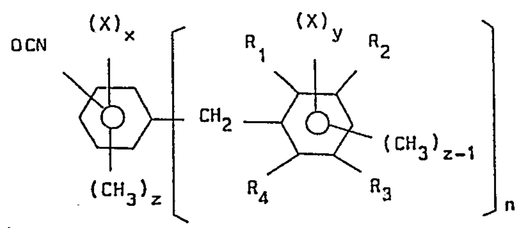

where

x = Cl, Br,
n = 1, 2,
x = 0, 1, 2,
y = 1, 2,
z = 1, 2.

One of the groups $R_1$, $R_2$, $R_3$, $R_4$ being an NCO group and the others being hydrogen atoms, depending on the values and positions of $(X)_y$ and $(CH_3)_{z-1}$.

2. Isocyanates according to Claim 1, characterised in that they are isomers of (isocyanatochlorobenzyl)toluene isocyanate.

3. Isocyanates according to Claim 1, characterised in that they are isomers of (isocyanatochlorobenzyl)chlorotoluene isocyanate.

4. Isocyanates according to Claim 1, characterised in that they are isomers of (isocyanato-chloro-para-methylbenzyl)chloro-para-xylene isocyanate.

5. Isocyanates according to Claim 1, characterised in that they are isomers of bis(isocyanato-chlorobenzyl)toluene isocyanate.

6. Isocyanates according to Claim 1, characterised in that they are mixtures of isomers of (isocyanatochlorobenzyl)toluene isocyanate and isomers of bis(isocyanatochlorobenzyl)toluene isocyanate.

7. Process of manufacture of halogenate aromatic isocyanates having an unsymmetrical diphenylmethane or polymethylphenylmethane structure according to Claims 1 to 6, characterised in that the compounds of formula:

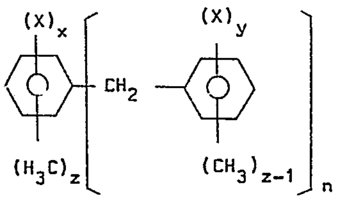

where X denotes a chlorine or bromine atom and where

x = 0 to 2,
y = 1 or 2,
z = 1 or 2,
n = 1 or 2,

are successively nitrated, hydrogenated and then phosgenated.

8. Process according to Claim 7, characterised in that the compounds having halogenated hydrocarbon structures are obtained by condensation of a halogenated benzyl chloride with toluene or xylene.

9. Process according to Claim 8, characterised in that the halogenated benzyl chloride is substituted with a methyl group.

10. Process according to Claim 8, characterised in that the toluene or xylene is chlorinated or brominated.

11. Process according to one of Claims 7 to 10, characterised in that the nitration is performed by means of a mixture of nitric acid and sulphuric acid of concentrations greater than 85%.

12. Process according to one of Claims 7 to 11, characterised in that the hydrogenation is performed in the presence of palladium as catalyst.